# EUROPEAN PATENT APPLICATION

(11) **EP 4 541 328 A1**
(43) Date of publication of application: **23.04.2025**
(21) Application number: 23203798.6
(22) Date of filing: 16.10.2023
(51) Int. Cl.: A61F 5/01, A61F 5/34

(54) **INFLATABLE COMPRESSION BRACE WITH HOT AND COLD COMPRESSES**

(71) Applicant: E-Life International Co., Ltd., New Tapei City (TW)
(72) Inventor: LEE, Shih-Hsiang, New Taipei City (TW)
(74) Representative: Casalonga

(57) **Abstract**

An inflatable compression brace with hot and cold compresses has a brace body (1), an air bag (3), and an inflate-and-deflate valve (4). The brace body wraps around joints and muscles. The air bag is formed within the brace body. The inflate-and-deflate valve is mounted on the brace body and connected to the air bag. The inflate-and-deflate valve can optionally inflate or deflate the air bag. Thereby the inflated air bag can compress and immobilize the targeted area. Additionally, when inappropriate pressure is applied to the target area, the inflate-and-deflate valve is capable of adjusting the air pressure.

## Description

### 1. Field of the Invention

The present invention relates to a medical equipment, especially to a medical brace.

### 2. Description of the Prior Arts

Joints and muscles in the human body are essential for movement, requiring the coordination of various joints and muscles to exercise. However, frequent use of joints and muscles can lead to muscle inflammation and strain, or damage to tissues, such as ligaments and tendons, which connects to muscles and joints. In post-injury treatment or rehabilitation, it's necessary to immobilize and compress the injured area, and in some cases, to apply cold or heat therapy.

The traditional protective brace is made from fabric that is wrapped around the injured area. To ensure stable fixation and applied pressure at a certain level, this protective brace is often layered around the injured area, using the fabric's elastic contraction to apply pressure. However, the process of wearing the protective brace is complicated and difficult for an individual to accomplish. Inappropriate pressure applied to the entire joint area might lead to poor blood circulation or discomfort. Adjustments to relieve discomfort typically require complete removal of the protective brace, making the process time-consuming and laborious.

To address the issues with traditional protective brace mentioned above, an inflatable brace has emerged in the existing technology. This inflatable brace consists of a brace body, an airbag, an inflation valve, and a deflation valve. The airbag is formed within the brace body and connected to the inflation valve and deflation valve. During inflation, an external inflation device is needed to inflate the airbag through the inflation valve, achieving the compression and immobilization. Since the inflation device is a separate external component from the inflatable brace, adjusting the pressure might be inconvenient, as the inflation device may not always be readily accessible.

The main objective of the present invention is to provide an inflatable compression brace with hot and cold compresses that is capable of compressing and immobilizing joints and muscles, thereby enhancing support and accelerating the healing process of the target area.

To achieve abovementioned purpose, the inflatable compression brace with hot and cold compresses includes a brace body, an air bag, and an inflate-and-deflate valve. The air bag is formed in the brace body. The inflate-and-deflate valve is mounted on the brace body and connected to the air bag. the inflate-and-deflate valve optionally inflates and deflates the air bag.

According to the abovementioned structure, the advantages of the inflatable compression brace with hot and cold compresses are that the inflatable airbag can compress and immobilize the target area, preventing the target area from swelling and secondary injuries. Moreover, when inappropriate pressure is applied to the target area, the user simply presses the first pressing component to adjust the air pressure, enhancing user convenience. As the inflate-and-deflate valve is mounted onto the brace body, there's no need for an external inflation device. The users can conveniently inflate and deflate the inflatable compression brace with hot and cold compresses in various scenarios.

### IN THE DRAWINGS

Fig. 1 is a front view of a first preferred embodiment in accordance with the present invention;
Fig. 2 is a back view of the first preferred embodiment in accordance with the present invention;
Fig. 3 is a schematic view showing the first preferred embodiment worn in accordance with the present invention;
Fig. 4 is a schematic view of the inflate-and-deflate valve in accordance with the present invention;
Fig. 5 is a cross sectional view of the inflate-and-deflate valve in accordance with the present invention;
Fig. 6 is a schematic view showing an inflating air bag in accordance with the present invention;
Fig. 7 is a schematic view of the inflating part recovering in accordance with the present invention;
Fig. 8 is a schematic view showing a deflating air bag in accordance with the present invention;
Fig. 9 is a front view of a second preferred embodiment in accordance with the present invention;
Fig. 10 is a back view of the second preferred embodiment in accordance with the present invention;
Fig. 11 is a schematic view showing the second preferred embodiment worn in accordance with the present invention;
Fig. 12 is a schematic view showing a third preferred embodiment worn in accordance with the present invention.

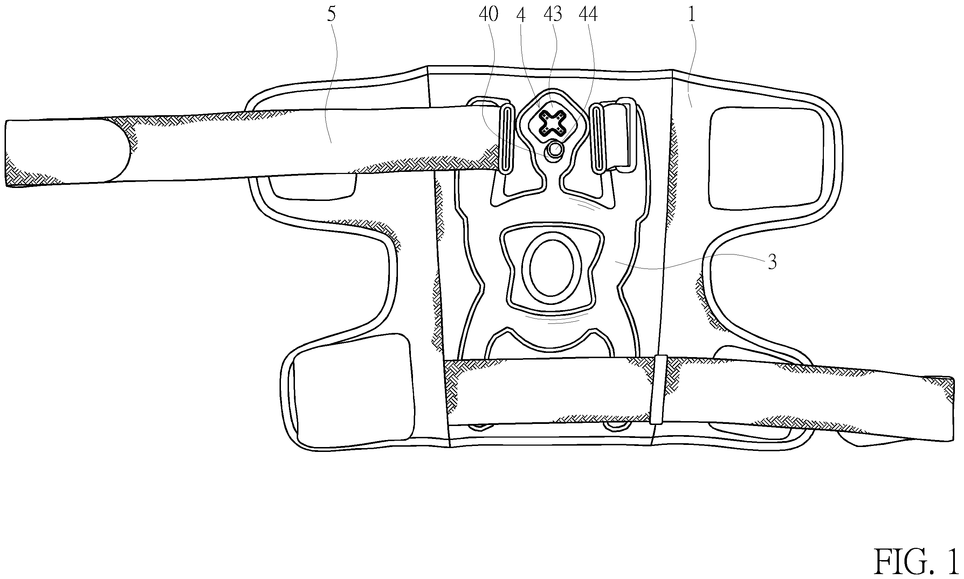

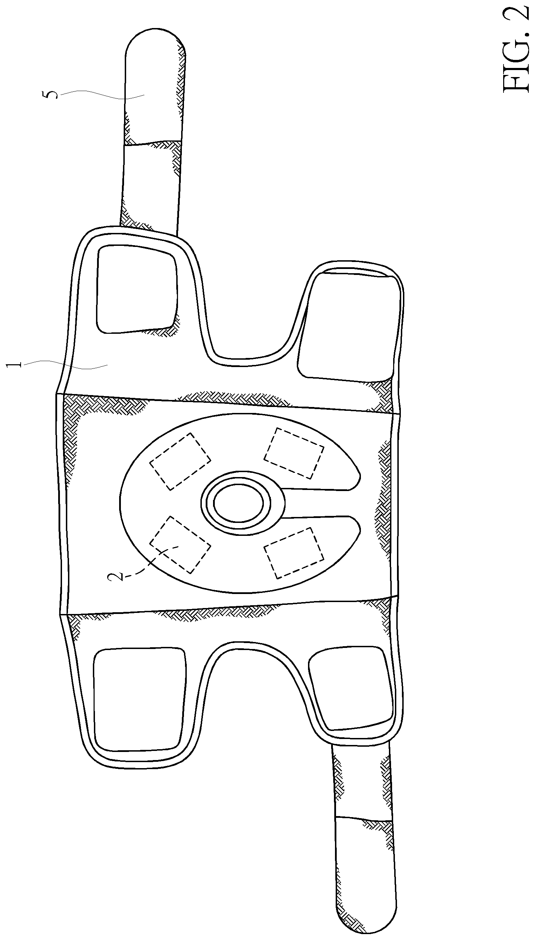

With reference to Figs. 1 and 2, an inflatable compression brace with hot and cold compresses in accordance with the present invention comprises a brace body 1, a compress 2, an air bag 3, an inflate-and-deflate valve 4, and a fastener component 5.

The brace body 1 is configured to wrap around joints or muscles, such as shoulder, waist, elbow, wrist, knee, etc., hereinafter called "the target area" in this specification. The compress 2 is detachably mounted in the brace body 1. In this embodiment, the brace body 1 has a pocket. The compress 2 can be accommodated within the pocket. In other embodiments, the compress 2 and the brace body 1 can be interlocked as hook and loop fasteners of Velcro, but it is not limited to this. The compress 2 includes a single-function cold compress or hot compress, or a multifunctional compress which can be cold or hot compress.

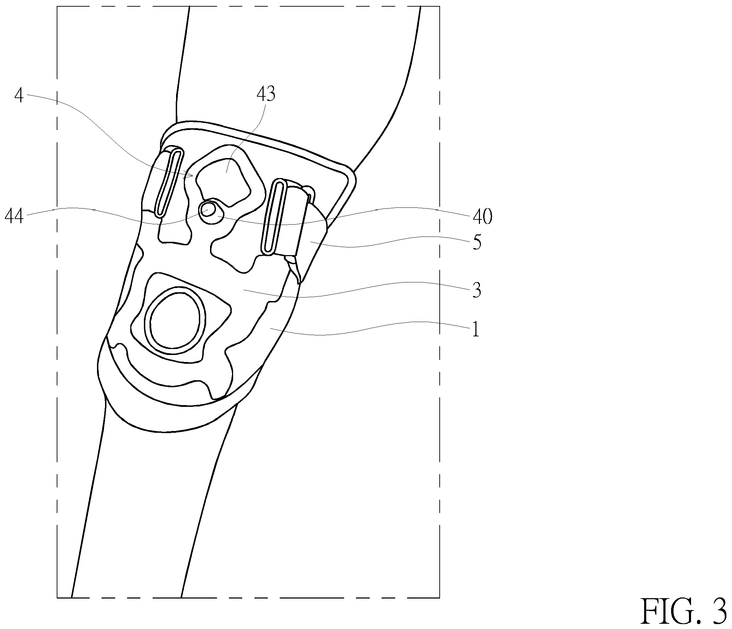

An air bag 3 is formed in the brace body 1. The shape and segmentation of the air bag3 vary according to the body part where the inflatable compression brace with hot and cold compresses is worn. With reference to Fig. 3, the first preferred embodiment is the inflatable compression brace with hot and cold compresses worn on the knee. The air bag 3 is located in the front of the knee. The air bag 3 resembles a strip around the kneecap, and extends toward thigh and calf. Thereby when the air bag 3 is inflated and pressurized, the air bag 3 can prevent direct compression on the kneecap while also enhancing the stabilization and support of the knee joint.

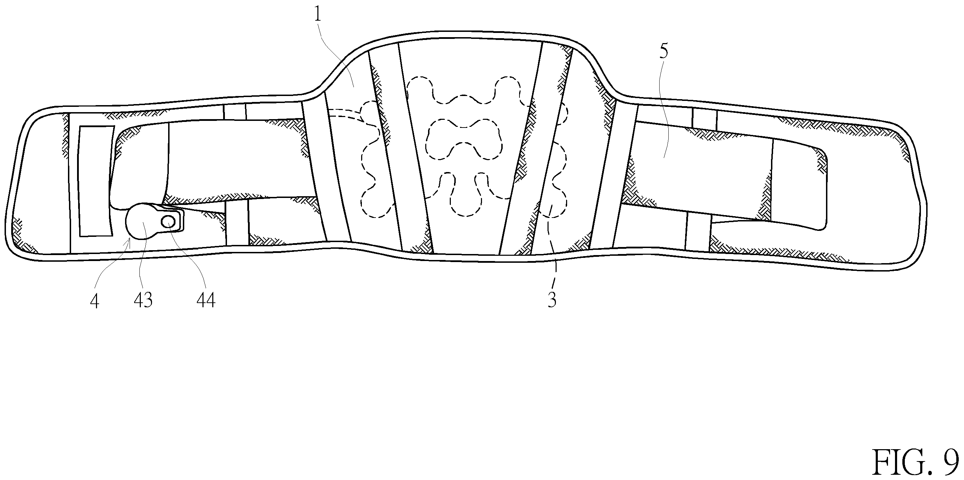

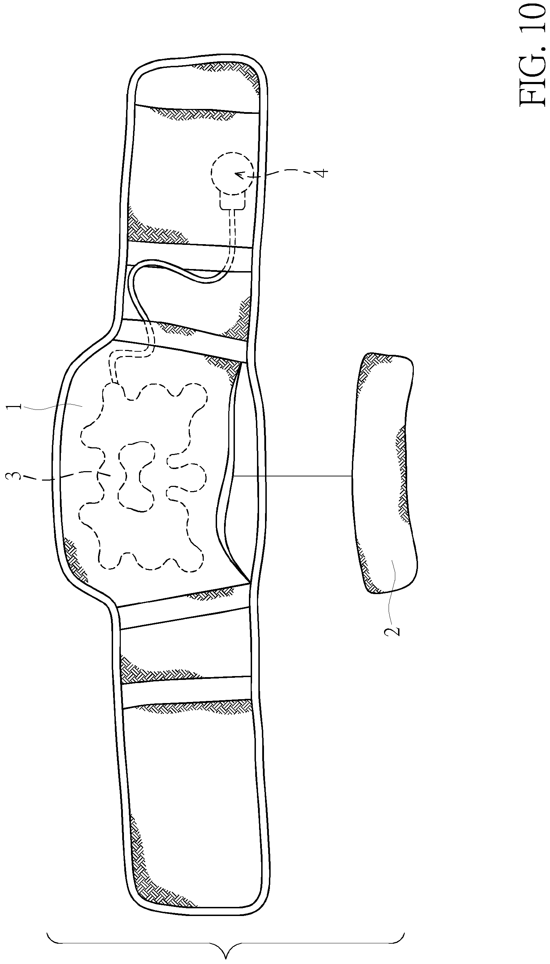

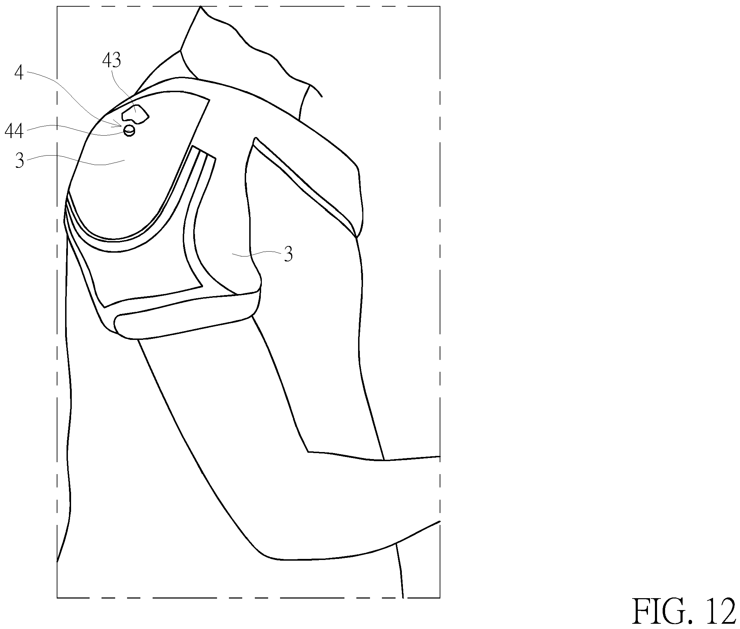

With reference to Figs. 9 to 11, the second preferred embodiment is the inflatable compression brace with hot and cold compresses worn on the waist. The air bag 3 is located on the back of the waist. The air bag 3 includes two sheet-like air bags respectively located on left and right sides with larger areas and the columnar air bag on center extending along a length direction of the spin. The columnar air bag is connected between the two sheet-like air bags. Therefore, when the air bag 3 is inflated and pressurized, the two sheet-like air bags on left and right sides can pressure and support the muscle group around the spin, while the columnar air bag on center supports the spin. With reference to Fig. 12, the second preferred embodiment is the inflatable compression brace with hot and cold compresses worn on the shoulder. The air bag 3 is located at the deltoid on the shoulder, in order to restrict the wearer from exercising over shoulder abduction.

With reference to Fig. 1, the inflate-and-deflate valve 4 is mounted on the brace body 1 and connected to the air bag 3. The inflate-and-deflate valve 4 can optionally inflate or deflate the air bag 3. When the air bag 3 is inflated, the brace body is less prone to bending or deformation due to activation of the wearer, thereby compressing and immobilize the target area, while ensuring a more complete fit of compress 2 onto the target area.

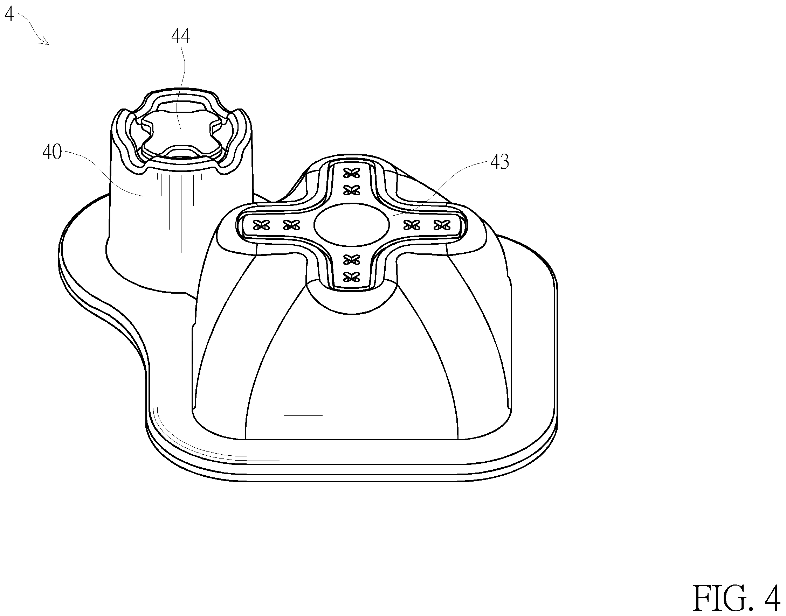

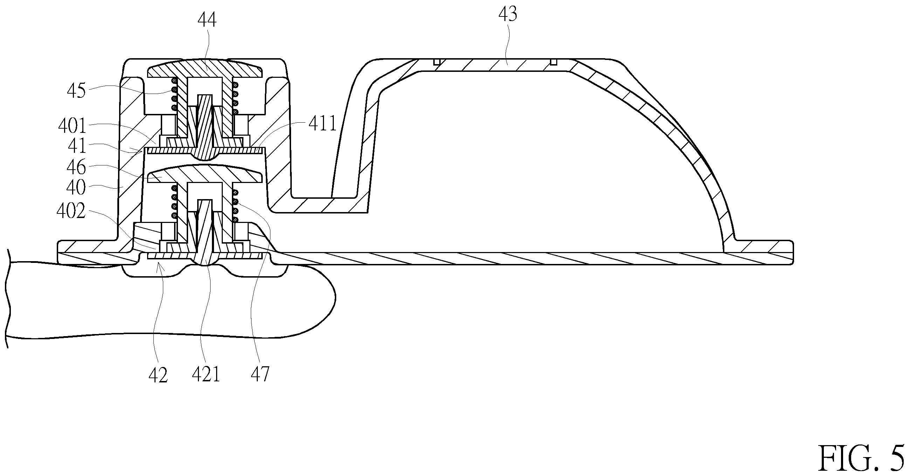

With reference to Figs. 4 and 5, the inflate-and-deflate valve 4 has a shell 40, a first check valve 41, a second check valve 42, an inflating part 43, a first compressing part 44, a first elastic component 45, a second compressing part 46, and a second elastic component 47.

The shell 40 forms internally an air space. The air space extends along with a first direction and connected to the air bag 3. The shell 40 has a first barrier 401 and a second barrier 402. The first barrier 401 surrounds and is mounted in the air space. The second barrier 402 surrounds and is mounted in the air space, and located at a distance from the first barrier 401 in the first direction.

The first check valve 41 is mounted in the air space, and includes a first sheet 411. In this embodiment, the first sheet 411 is movable, thereby optionally resting against or separating from the first barrier 401. When the first check valve 41 moves along the first direction, the first sheet 411 separates from the first barrier 401, enabling air to flow through the first check valve 41. In other embodiments, the first sheet 411 is immovable, but may bend or deform due to changes in air pressure or airflow. For instance, if the pressure within the air space becomes lower than the external air pressure, the first sheet 411 may bend or deform, causing it to separate from the first barrier 401, thereby enabling air to flow through the first check valve 41.

The second check valve 42 is mounted in the air space, and located at a distance from the first check valve 41 in the first direction. The second check valve 42 includes the second sheet 421. In this embodiment, the second sheet 421 is movable, thereby optionally resting against or separating from the second barrier 402. When the second check valve 42 moves along the first direction, the second sheet 421 separates from the first barrier 401, enabling air to flow through the second check valve 42. In other embodiments, the second sheet 421 is immovable, but may bend or deform due to changes in air pressure or airflow. For instance, if the pressure within the air space becomes lower than the external air pressure, the second sheet 421 may bend or deform, causing it to separate from the second barrier 402, thereby enabling air to flow through the second check valve 42.

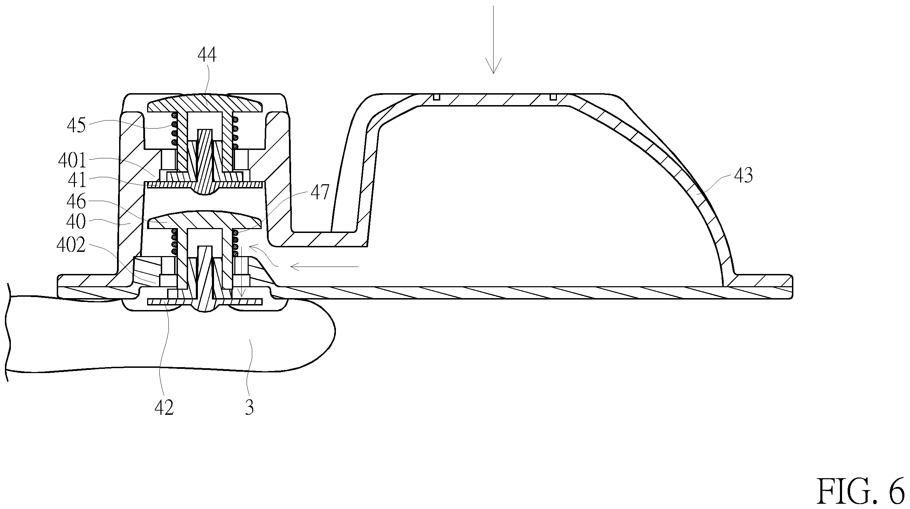

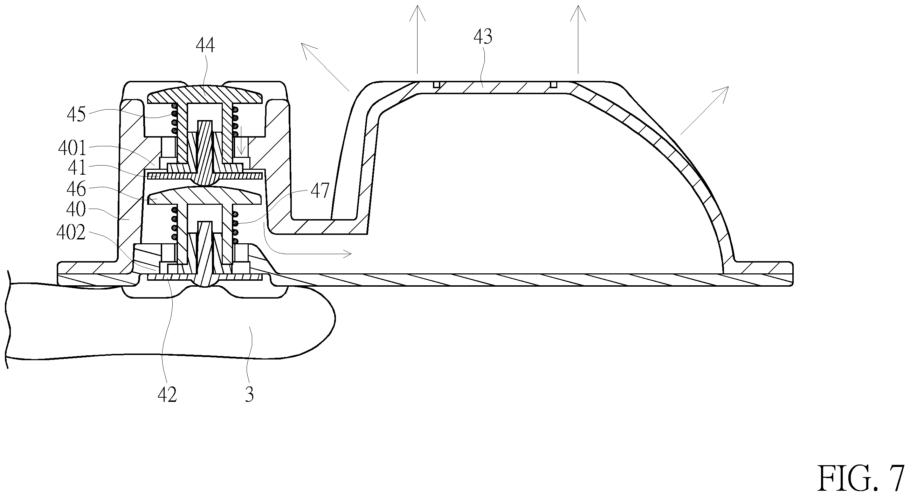

The inflating part is a hollow shell and is connected to the air space between the first check valve 41 and the second check valve 42. With reference Fig. 6, when the inflating part 43 is subjected to a squeeze, the inflating part 43 will be squashed and push air in the hollow shell to the air space, and air will separate the second check valve 42 from the second barrier 402 to enter the air bag 3. The inflating part 43 consists of highly elastic materials. With reference Fig. 7, when the squeeze is released, the inflating part 43 squashed will bounce back to its original state with the elastic resilience. At the same time, the air pressure in the hollow sell of the inflating part 43 is lower than atmospheric pressure, enabling air at outside environment to flow into the air space and separate the first check valve 41 from the first barrier 401, in order to re-inflate the inflating part 43 again. Therefore, the user can inflate the air bag 3 by repeating pressing the inflating part 43.

With reference Fig. 5 to 8, the first compressing part 44 is connected to the first check valve 41. The first compressing part 44 can drive the first check valve 41 to move along the first direction. The first elastic component 45 is connected to the first compressing part 44, and pushes the first compressing part 44 with resilience in a direction opposite of the first direction.

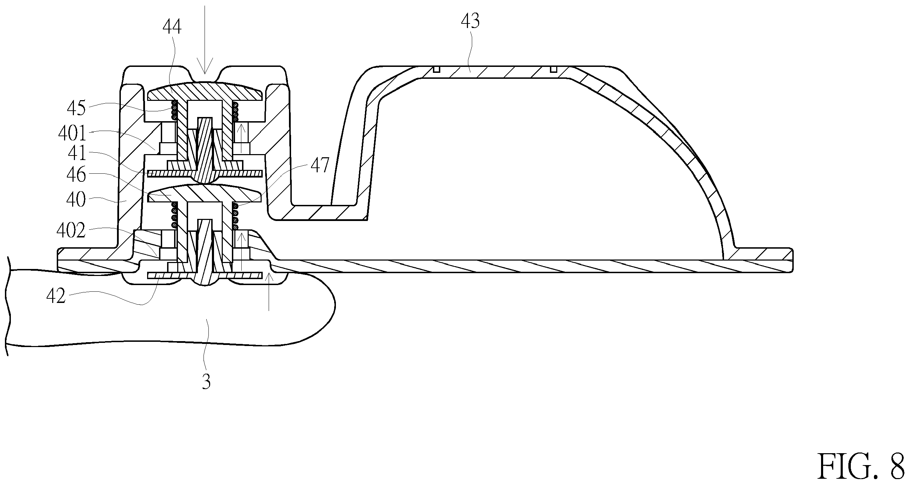

With reference Fig. 8, when the air bag 3 is inflated and the user presses the first compressing part 44 to drive the first check valve 41 to move along the first direction, the first check valve 41 will separate from the first barrier 401 and push the second compressing part 46 to move along the first direction to drive the second check valve 42 to separate from the second barrier 402, thereby connecting the air bag 3, the air space, and outside environment and enabling air in the air bag 3 to flow out of the air bag 3 after compressing. When worn, the inflatable compression brace with hot and cold compresses applies a reaction force by the target area. Therefore, the user only needs to keep pressing the first compressing part 44 to achieve the deflation.

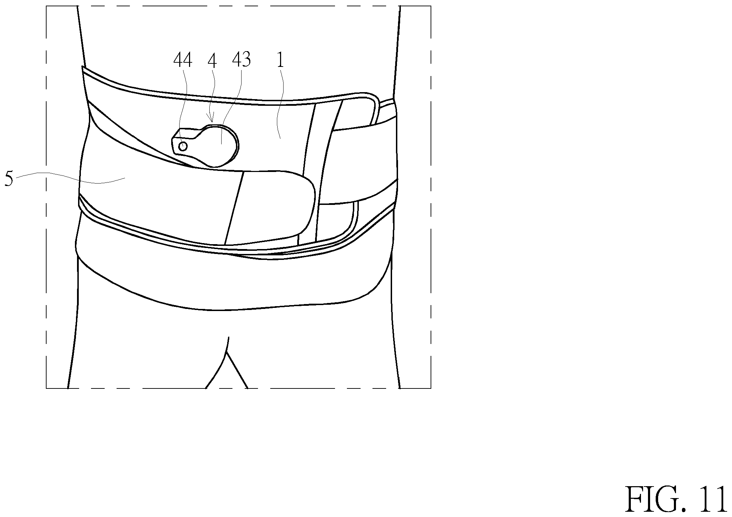

With reference Fig. 3 and 11, the fastener component 5 is an elastic band, and is capable of wrapping around the brace body 1. In this embodiment, the end of the fastener component 5 and the strap body of the fastener component 5 can be interlocked as hook and loop fasteners of Velcro, or the end of the fastener component 5 and the brace body 1 can be interlocked as hook and loop fasteners of Velcro. The fastener component 5 can compress to the target area by wrapping around the target area with its own elasticity, and the end of the fastener component 5 adheres to either its own strap body or the brace body 1. Thereby the inflatable compression brace with hot and cold compresses more securely and stably wraps around the target area.

With reference Fig. 5 to 8, when wearing the inflatable compression brace with hot and cold compresses, the user wraps the brace body 1 around the target area first, and then repeats pressing the inflating part 43 to inflate the air bag 3. When adjusting the air pressure of the inflatable compression brace with hot and cold compresses, the user keeps pressing the first compressing part 44. At this moment, the air bag 3 will continue to release air. When the appropriate air pressure is reached, the user can stop pressing the first compressing part 44.

The present invention is not limited to the first preferred embodiment, the second preferred embodiment, and the third preferred embodiment. In other embodiments, it can be applied to more target areas such as wrists, elbows, ankles, neck, etc. The inflate-and-deflate valve 4 is mounted in a location that is convenient for user operation, and the shape and segmentation of air bag 3 vary according to the muscle distribution or skeletal structure of the target area.

The advantage of the inflatable compression brace with hot and cold compresses is that the inflated air bag 3 can apply pressure and fixation to the target area to prevent the target area from swelling and secondary injuries. Moreover, the inflatable compression brace with hot and cold compresses can make the compress 2 fit more snugly on the target area, achieving better temperature transfer to the target area. When inappropriate pressure is applied to the target area, the user can simply press the first compressing part 44 to adjust the air pressure, thereby enhancing user convenience. As the inflate-and-deflate valve 4 is mounted on the brace body 1, there is no need for an external inflation device, allowing inflation and deflation of the inflatable compression brace with hot and cold compresses to be easily done in various scenarios.

## Claims

1. An inflatable compression brace with hot and cold compresses **characterized in that** the inflatable compression brace with hot and cold compresses comprises:
a brace body (1);
an air bag (3) formed in the brace body (1);
an inflate-and-deflate valve (4) mounted on the brace body (1) and connected to the air bag (3); the inflate-and-deflate valve (4) optionally inflating and deflating the air bag (3).

2. The inflatable compression brace with hot and cold compresses as claimed in claim 1, wherein
the inflate-and-deflate valve (4) has:
a shell (40) forming an air space and communicating with the air bag (3);
a first check valve (41) mounted in the air space;
a second check valve (42) mounted in the air space at a distance from the first check valve (41) in a first direction;
an inflating part (43) being a hollow shell (40) and connected to the air space between the first check valve (41) and the second check valve (42);
wherein when the first check valve (41) moves along the first direction, the air space fluidly communicates with an environment, allowing air to flow through the first check valve (41);
when the second check valve (42) moves along the first direction, the air space fluidly communicates with the air bag (3), allowing air to flow through the second check valve (42);
when the inflating part (43) is subjected to a squeeze and thereby air in the inflating part (43) is pushed into the air space, air flows through the second check valve (42) to enter the air bag (3), when the squeeze is released, air at the environment flows through the first check valve (41) to enter the inflating part (43).

3. The inflatable compression brace with hot and cold compresses as claimed in claim 2, wherein,
the inflate-and-deflate valve (4) has:
a first compressing part (44) connected to the first check valve (41); the first compressing part (44) being capable of driving the first check valve (41) to move along the first direction;
a first elastic component (45) connected to the first compressing part (44), and pushing the first compressing part (44) in an opposite direction of the first direction with resilience;
a second compressing part (46) connected to the second check valve (42); the second compressing part (46) being capable of driving the second check valve (42) to move along the first direction;
a second elastic component (47) connected to the second compressing part (46), and pushing the second compressing part (46) in the opposite direction of the first direction with resilience;
wherein when the first compressing part (44) drives the first check valve (41) to move along the first direction, the first check valve (41) pushes the second compressing part (46) and the second check valve (42) to move along the first direction.

4. The inflatable compression brace with hot and cold compresses as claimed in claim 2 or 3, wherein,
the shell (40) has:
a first barrier (401) surrounding and mounted in the air space;
a second barrier (402) surrounding and mounted in the air space, and located at a distance from the first barrier (401) in the first direction;
the first check valve (41) includes a sheet and optionally abuts or separates from the first barrier (401); when the first check valve (41) moves along the first direction, the first check valve (41) separates from the first barrier (401), and air flows through the first check valve (41);
the second check valve (42) includes a sheet and optionally abuts or separates from the second barrier (402); when the second check valve (42) moves along the first direction, the second check valve (42) separates from the second barrier (402), and air flows through the second check valve (42).

5. The inflatable compression brace with hot and cold compresses as claimed in claim 1, wherein the inflatable compression brace with hot and cold compresses has:
a compress (2) detachably mounted in the brace body (1).

6. The inflatable compression brace with hot and cold compresses as claimed in claim 5, wherein the inflatable compression brace with hot and cold compresses has a pocket; the compress (2) is detachably mounted within the pocket.

7. The inflatable compression brace with hot and cold compresses as claimed in claim 5, wherein the compress (2) and the brace body (1) are interlocked as a couple of hook and loop fasteners.

8. The inflatable compression brace with hot and cold compresses as claimed in claim 1, wherein the inflatable compression brace with hot and cold compresses has:
a fastener component (5) being capable of wrapping around the brace body (1).

9. The inflatable compression brace with hot and cold compresses as claimed in claim 8, wherein the fastener component (5) is an elastic band.

10. The inflatable compression brace with hot and cold compresses as claimed in claim 8 or 9, wherein the fastener component (5) has:
an end;
a strap body connected to the end;
wherein the end and the strap body are configured to interlock as a couple of hook and loop fasteners, or the end and the brace body (1) are configured to interlock as a couple of hook and loop fasteners.
